# EUROPÄISCHE PATENTSCHRIFT

(11) **EP 3 703 571 B1**
(45) Veröffentlichungstag und Bekanntmachung des Hinweises auf die Patenterteilung: **15.09.2021**
(21) Anmeldenummer: 18793634.9
(22) Anmeldetag: 24.10.2018
(51) Int. Cl.: A61B 6/14, G03B 42/04, A61C 5/80, B29C 64/00

(54) **HALTEVORRICHTUNG FÜR RÖNTGENFILME**
DEVICE FOR HOLDING X-RAY FILMS
DISPOSITIF DE RETENUE POUR FILMS RADIOGRAPHIQUES

(30) Priorität: 03.11.2017 DE 102017125671
(43) Veröffentlichungstag der Anmeldung: 09.09.2020
(73) Patentinhaber: SICAT GmbH & Co. KG, 53175 Bonn (DE)
(72) Erfinder: ZOLLORSCH, Andreas, 53177 Bonn (DE); CIZEK, Jiri, 85748 Garching b. München (DE)
(74) Vertreter: Braun-Dullaeus Pannen Emmerling Patent- & Rechtsanwaltspartnerschaft mbB
(86) Internationale Anmeldenummer: PCT/EP2018/079106
(87) Internationale Veröffentlichungsnummer: WO 2019/086302

(56) Entgegenhaltungen:
- JP-A- H05 317 307
- US-A- 5 090 047
- US-A1- 2015 359 479

## Beschreibung

Die Erfindung betrifft eine Haltevorrichtung für Röntgenfilme sowie ein Verfahren zur Herstellung der Haltevorrichtung für Röntgenfilme wie sie insbesondere bei zahnmedizinischen Behandlungen durch einen Zahnarzt eingesetzt werden. Hierbei wird ein Röntgenfilm während der Erstellung einer bildgebenden Diagnose von der Haltevorrichtung im Mundraum gehalten. Unter einem Röntgenfilm ist im Rahmen dieser Erfindung auch ein digitaler Sensor erfasst, die häufig anstelle der "klassischen" Röntgenfilme in der Zahntechnik Verwendung finden.

Im Alltag von Zahnarztpraxen werden häufig Röntgenaufnahmen von den Zähnen bzw. des Kiefers eines Patienten aufgenommen, damit der Zahnarzt eine Diagnose stellen kann. Eine Zahnarzthelferin platziert hierbei den Röntgenfilm in dem Mundraum des Patienten bevor die bildgebende Diagnostik gestartet wird. Sind mehrere Bildaufnahmen im Laufe der Behandlung von dem Zahn bzw. vom Kiefers nötig, müssen mehrere Röntgenaufnahmen auf verschiedenen Röntgenfilmen angefertigt werden, wobei der Arzt den Verlauf der Behandlung bzw. des Krankheitsverlaufs aus dem Vergleich der verschiedenen Röntgenfilme ersehen kann. Nachteilig wird dem Arzt die Diagnose dahingehend erschwert, dass die Röntgenfilme nur beschränkt vergleichbar sind, da ihre Position von Aufnahme zu Aufnahme stark variieren kann. Weder bringen die Zahnarzthelferin die Röntgenfilme bei jeder Aufnahme an der gleichen Stelle in dem Mundraum ein noch sind die Röntgenfilme an dieser Stelle fixiert, sodass die Röntgenfilme vor und sogar während der Röntgenaufnahme sowohl ihre Translation- als auch ihre Rotationskoordinaten ändern können. Das Ergebnis der Befunde kann also beispielsweise dadurch verfälscht werden, dass ein Zahn bei verschiedenen Röntgenfilmen unter verschiedenen Blickwinkeln betrachtet wird, die eine Vergleichbarkeit erschweren. Insgesamt ergibt sich also eine Variabilität der Ausgangsparameter.

US 7,204,640 B2 zeigt eine Vorrichtung und ein Verfahren, die es ermöglichen Röntgenfilme mit einen digitalen volumentomographischen (DVT) Datensatzes zu registrieren, um bestimmen zu können, wie der Röntgenfilm innerhalb des DVT-Datensatzes angeordnet ist. Dieses Verfahren findet beispielsweise bei der Patientenpositionierung in der Strahlentherapie Anwendung. Die Behandlung wird zumeist mittels eine DVT-Datensatzes geplant, beispielsweise durch eine Dosisverteilung bei einer Tumorbestrahlung und der Patient wird dann anhand einer 2D-Röntgenaufnahmen, welche unmittelbar vor der Behandlung angefertigt wird, positioniert. Um den Patienten an die richtige Stelle für die Bestrahlung positionieren zu können, muss ermittelt werden, wo sich die 2D-Röntgenaufnahmen innerhalb des DVT-Datensatzes befindet.

In diesem Zusammenhang offenbaren die US 2015/359479 A1 und die US 5 090 047 A ebenfalls die Herstellung einer Haltevorrichtung für Röntgenfilme, wobei die Haltevorrichtung an vorhandene Zähne anbringbar ist.

Das Platzieren eines Röntgenfilms bzw. eines Röntgensensors im Mundraum des Patienten ist mit einigen Schwierigkeiten behaftet, da insbesondere die Positionierung des Sensors mit vielen Freiheitsgraden versehen ist: Die Position im Kieferbogen, die Rotation des Sensors um seine Achsen und das Aufbeisen des Patienten auf den Sensorhalter bzw. Filmhalter verändern die Lage des Sensors auf Basis von spezifischen Gebissstellungen, Unter- bzw. Überbiss und Beißdruck des Patienten. Um also ein Röntgenbild mittels eines Intraoralsensors reproduzierbar erzeugen zu können, sodass eine bessere Vergleichbarkeit erzielt werden kann, müssen alle diese Unsicherheitsfaktoren unter Kontrolle gehalten werden.

Der vorliegenden Erfindung liegt daher die Aufgabe zugrunde ein Verfahren sowie eine Vorrichtung anzugeben, die es ermöglichen verschiedene Röntgenfilme so aufzunehmen, dass diese mit gleichen Ausgangsparametern erstellt werden und deshalb vergleichbar sind.

Diese Aufgabe wird gelöst durch die unabhängigen Ansprüche. Vorteilhafte Ausführungsformen sind in den jeweiligen Unteransprüchen genannt. Erfindungsgemäß wird bei dem Verfahren zur Herstellung einer Haltevorrichtung für Röntgenfilme eines Patienten mittels eines Rechners eine Bildregistrierung eines ersten, insbesondere intraoralen, Röntgenbildes eines Zahns des Unter- oder Oberkiefers in einem digitalen dreidimensionalen volumentomographischen Datensatz des Mundraums durchgeführt, wobei die Lage des ersten Röntgenbildes innerhalb des volumentomographischen Datensatzes bestimmt wird und anhand des volumentomographischen Datensatzes ein virtuelles Modell für eine an den Zähnen des Unter- oder Oberkiefers formschlüssig anbringbare Haltevorrichtung erstellt wird, die ein Halteelement für die Halterung eines Röntgenfilms in definierter Position aufweist, wobei die definierte Position durch die Lage des ersten Röntgenbilds im volumentomographischen Datensatz festgelegt wird und die Haltevorrichtung entsprechend dem virtuellen Modell hergestellt wird. Unter einem Röntgenfilm ist im Rahmen dieser Erfindung auch ein digitaler Sensor erfasst, die häufig anstelle der "klassischen" Röntgenfilme in der Zahntechnik Verwendung finden. Um die Qualität bzw. die Auflösung des volumentomographischen Datensatz weiter zu erhöhen, können optional zudem digitale Zahnabdrücke, welche mit der CAD/CAM CEREC Methode erstellt wurden, mit dem volumentomographischen Datensatz fusioniert werden. Bei der CAD/CAM CEREC Methode wird mittels einer intraoralen Kamera ein optischer Abdruck des Zahns oder der Zähne eingescannt und ein dreidimensionales Modell errechnet. Dieses kann auf dem Monitor dargestellt und bearbeitet und mit dem volumentomographischen Datensatz fusioniert werden.

Vorteilhaft wird durch die Registrierung des ersten intraoralen Röntgenbildes eines ersten Röntgenfilms mit dem volumentomographischen Datensatz erreicht, dass die Lage des ersten intraoralen Röntgenbildes innerhalb des volumentomographischen Datensatzes bekannt ist. Innerhalb des virtuellen Modells ist das Halteelement an dieser definierten Position vorgesehen, sodass ein zweiter Röntgenfilm, der in das Halteelement eingebracht bzw. von diesem gehalten wird, bei der Bildgebung an derselben Position gehalten wird wie der erste Röntgenfilm. Da die Haltevorrichtung zudem formschlüssig an den Zähnen angebracht wird, ist sichergestellt, dass sich die Haltevorrichtung bei der Bildgebung nicht in ihrer Position ändert. Der erste Röntgenfilm kann also wie herkömmlich von einem Zahnarzt erstellt werden, wobei dieser auf einfache Weise mit jedem weiteren Röntgenfilm, der von dem Halteelement gehalten wird, verglichen werden kann. Um die Formschlüssigkeit des virtuellen Modells zu berechnen werden wahlweise der volumentomographische Datensatz oder alternativ ein Gipsmodell verwendet, welches anschließend digitalisiert und mit dem volumentomographischen Datensatz fusioniert wird. Eine alternative Bezeichnung für die Haltevorrichtung ist eine Schablone bzw. Zahnschablone oder Bohrschablone. Mit dem Begriff Schablone wird der Tatsache Rechnung getragen, dass die Haltevorrichtung, also die Schablone, zugleich für weitere zahnmedizinische Eingriffe verwendet werden kann, da zusätzlich zu dem Halteelement beispielsweise ein Bohrkanal vorgesehen sein kann. Bevorzugt läuft die Registrierung automatisch bzw. semi-automatisch ab. Dies kann auch so verstanden werden, dass beim Anfertigen einer Bohrschablone die Haltevorrichtung quasi zusätzlich ohne großen weiteren Aufwand erstellt werden kann.

Vorzugsweise wird die Haltevorrichtung mittels einer Frästechnik und/oder im 3D-Druck anhand des virtuellen Modells und der definierten Position des Halteelements im volumentomographischen Datensatz hergestellt. Beide Herstellungsverfahren ermöglichen eine präzise, reproduzierbare und kostengünstige Herstellung der Haltevorrichtung.

In einer bevorzugten Ausführung der Erfindung ist die Haltevorrichtung aus biokompatiblen Material gefertigt, da die Haltevorrichtung im Mundraum des Patienten verwendet wird und im Idealfall auch natürlich abbaubar sein soll. Das Material soll möglichst nicht opak bezüglich Röntgenstrahlung sein.

Bevorzugt ist die Haltevorrichtung, insbesondere das Halteelement, aus einem homogenen Material mit möglichst geringer Ordnungszahl hergestellt. Homogene Eigenschaften gewährleisten, dass die Röntgenstrahlung auf jeder Stelle des Films mit gleicher Intensität auftrifft und dadurch die Ergebnisse nicht verfälscht werden. Die Abschwächung von Röntgenstrahlen beim Durchqueren von Materie ist materialabhängig und wird durch das Lambert-Beersche Gesetz beschrieben: I = I₀*exp(-k*d), wobei I₀ die Anfangsintensität, d die Wegstrecke und k ein materialabhängiger Koeffizient ist, welcher proportional zur vierten Potenz der Ordnungszahl des Materials ist, das von der Röntgenstrahlung durchquert wird. Vorzugsweise sollte das Material also eine Ordnungszahl haben, welche insbesondere kleiner als Kalzium ist, da Kalzium der primäre Wechselwirkungspartner der Röntgenstrahlung im menschlichen Körper ist. Andernfalls müsste die Dosis der Röntgenstrahlung nachteilig für den Patienten stark erhöht werden, um für den zweiten Röntgenfilm dieselbe Schwärzung wie beim ersten Röntgenfilm zu erhalten. Geeignet sind Materialien, die sich aus Kohlenwasserstoffverbindungen herstellen lassen.

Vorteilhaft ist das Halteelement in einer Durchstrahlrichtung, insbesondere an den Stellen, die die zu untersuchenden Stellen im Mundraum verdecken, der Röntgenstrahlung möglichst dünn ausgebildet. Die Materialdicke des Halteelements ist an in der Durchstrahlrichtung dünner als 1 mm, bevorzugt dünner als 0,75 mm und besonders bevorzugt dünner als 0,5 mm. An Stellen, die das Halteelement mit der Haltevorrichtung verbindet, ist die Materialstärke so vorzusehen, dass das Halteelement möglichst starr und unbeweglich mit der Haltevorrichtung verbunden, sodass sich die definierte Position nicht verändert. An diesen Stellen ist bevorzugt eine Materialdicke von mehr als 1 mm, bevorzugt von mehr als 3 mm vorgesehen. Auch an anderen Stellen, insbesondere denen, die zur Fixierung des Röntgenfilms dienen, kann eine dickere Materialstärke des Halteelements notwendig sein. An jenen Abschnitten des Halteelements, die die zu untersuchenden Stellen der Zähne bzw. des Kiefers verdecken, ist eine möglichst dünne Materialstärke geeignet, da sonst, wie vorstehend erläutert, die Dosis der Röntgenstrahlung erhöht werden müsste, um dieselben Schwärzungsgrade der Röntgenfilme wie ohne das Halteelement zu erzielen. Das Halteelement ist hierbei in der Art einer Tasche vorgesehen, in die der Röntgenfilm von oben einschiebbar ist.

Alternativ kann das Halteelement in der Durchstrahlrichtung für Röntgenstrahlung, insbesondere an den Stellen, die die zu untersuchenden Stellen im Mundraum verdecken, in einer U-Form ausgebildet sein. Hierbei können in die innenliegenden Flächen der U-förmigen Form des Halteelements Nut-artige Vertiefungen eingearbeitet sein in die der Röntgenfilm von oben einschiebbar ist. Das Halteelement ist hierbei in der Art eines Rahmens vorgesehen. In dieser Ausführung wird die Röntgenstrahlung nicht abgeschwächt, da die zu untersuchenden Stellen der Zähne bzw. des Kiefers nicht von Material des Halteelements verdeckt werden. Dadurch, dass der Röntgenfilm von oben eingeschoben wird, ist dieser in der U-förmigen Form gegenüber der Gravitationskraft fixiert.

Alternativ umfasst das Halteelement Haltehaken, die an strategischen Positionen angebracht werden, um den Röntgenfilm zu halten. Je nach Anwendungsfall kann der Röntgenfilm mittels dieser Haltehaken eingespannt werden, sodass zusätzliche Freiheitsgrade z.B. Verschiebung der Projektionsfläche noch möglich sind, um beispielsweise die Wurzel besser zu erkennen.

Bevorzugt werden die geometrischen Abmessungen des Röntgenfilms bei der Herstellung des Halteelements eingerechnet. Röntgenfilme von verschiedenen Herstellern können verschiedene geometrische Dimensionen aufweisen, welche bei der Erstellung des virtuellen Modells und der Fertigung des Halteelements berücksichtigt werden müssen, damit der entsprechende Röntgenfilm so an das Halteelement bzw. in das Halteelement eingebracht werden kann, dass sich dessen Lage bei der Bildgebung nicht verändert, was die Vergleichbarkeit der Röntgenfilme wiederum nachteilig beeinflussen würde.

Gemäß einem weiteren Aspekt der Erfindung ist eine Haltevorrichtung angegeben, die nach den vorstehend beschriebenen Verfahrensmerkmalen hergestellt ist.

Bevorzugt ist der Röntgenfilm formschlüssig in das Halteelement einsteckbar. Hierdurch wird gewährleistet, dass der Röntgenfilm keinen Bewegungsspielraum hat und in seiner definierten Position verbleibt.

Vorzugsweise weist die Haltevorrichtung einen Zahnaufsatz in Form einer Zahnschablone auf. Hierdurch kann die Haltevorrichtung einfach auf die Zähne des Patienten aufgesetzt werden. Da der Zahnaufsatz an die Oberfläche der Zähne des Patienten angepasst ist, wird durch das Aufsetzen eine lösbare Fixierung der Haltevorrichtung erreicht.

In einer bevorzugten Ausgestaltung der Erfindung ist der Zahnaufsatz mittels eines Brückenelements mit dem Halteelement verbunden. Das Brückenelement ermöglicht es vorteilhaft, dass das Halteelement nicht direkt in den "Körper" der Haltevorrichtung eingearbeitet werden muss, sondern auch beabstandet zu diesem angeordnet sein kann. Hierdurch wird es leichter möglich sowohl die definierte Position zu realisieren als auch den Röntgenfilm in das Halteelement einzubringen. Insbesondere ist das Brückenelement für die U-Form des Halteelements vorteilhaft.

Weitere Vorteile, Eigenschaften und Weiterbildungen der Erfindung ergeben sich aus den abhängigen Ansprüchen sowie aus der nachstehenden Beschreibung von bevorzugten Ausführungsbeispielen.

Die Erfindung wird nachstehend unter Bezugnahme auf die anliegenden Zeichnungen anhand bevorzugte Ausführungsbeispiele näher erläutert.
- Fig. 1: zeigt ein erstes schematisches Ausführungsbeispiel einer erfindungsgemäßen Bohrschablone mit einem Halteelement in einer Schnittdarstellung.
- Fig. 2: zeigt ein zweites schematisches Ausführungsbeispiel der erfindungsgemäßen Bohrschablone aus Fig. 1.
- Fig. 3: zeigt ein erstes Ausführungsbeispiel des Halteelements aus Fig. 1 in Form einer Tasche zur Aufnahme von Röntgenfilmen in perspektivischer Darstellung.
- Fig. 4a: zeigt ein zweites Ausführungsbeispiel des Halteelements aus Fig. 3 in Form eines U-förmigen Rahmens in einem Querschnitt entlang einer Durchstrahlrichtung der Röntgenstrahlung.
- Fig. 4b: zeigt das Halteelement in Schnitt durch die Ebene A aus Fig. 4a.
- Fig. 5a: zeigt ein drittes Ausführungsbeispiel des Halteelements aus Fig. 3 in Form einzelner Haltehaken entlang einer Durchstrahlrichtung der Röntgenstrahlung.
- Fig. 5b: zeigt das Halteelement in einer Draufsicht durch die Ebene A aus Fig. 5a.

Das Verfahren, welches zur Herstellung der Haltevorrichtung führt, kann wie folgt ablaufen:
Ein Patient kommt mit einer Zahnwurzelentzündung und einem intraoral-Röntgenbild von einem überweisenden Arzt zu einem Facharzt. Der Facharzt will den Zahn 20 bzw. den Kiefer genauer untersuchen und fertigt ein DVT-Datensatz an, um eine endodontologische Planung vorzunehmen und eine Therapie- /Bohrschablone 26 für diese Planung zu bestellen. Zusätzlich lädt der Facharzt das intraorale-Röntgenbild zusammen mit dem DVT-Datensatz in eine Software und registriert das interorale-Röntgenbild mittels eines (semi-) automatischen Algorithmus an die Stelle im DVT-Datensatz an der der Röntgenfilm lag als die Bildaufnahme des Röntgenfilms getätigt wurde. Abgesehen von einer präziseren Befundung, die durch die Fusionierung des intraoralen-Röntgenbildes und des DVT-Datensatzes möglich ist, verfügt der Facharzt/Spezialist bzw. die Software nun über die Ortsdaten des Röntgenfilms im Bezug zu dem Kopf des Patienten.

Um eine Bohrschablone 26 zu bestellen, macht der Facharzt noch eine digitale Gebissabformung des Kiefers, der den zu behandelnden Zahn 20 trägt. Alternativ kann die Form des Gebisses auch direkt aus dem DVT-Datensatz extrahiert werden. Die Gebissabformung registriert der Facharzt ebenfalls mit dem DVT-Datensatz des Patienten. Der Facharzt bestellt die Bohrschablone 26 und schickt die Registrierungsdaten (Röntgenfilm und Gebissabformung) sowie die dazugehörigen Röntgen- und Oberflächendaten an ein Labor. Das Labor erstellt die zahngetragene Bohrschablone 26, welche vorstehend auch als Haltevorrichtung 26 bezeichnet wurde, für die Behandlung. Das Labor bringt an die zahntragende Bohrschablone 26 zusätzlich noch ein Halteelement 30 in Form einer Tasche 30 an, in die der Röntgenfilm 38 eingebracht werden kann. Die Position der Tasche 30 ist derart vorgesehen, dass sich der zweite eingesteckte Röntgenfilm 38, wenn der Patient die Schablone auf den dafür vorgesehene Zähnen 20 trägt, an der gleichen Stelle befindet, an der sich der erste Röntgenfilm befand mit dem das ursprünglich erste intraorale-Röntgenbild erstellt wurde.

Nach der Behandlung macht der Arzt ein zweites intraorales-Röntgenbild des behandelten Zahns 20, indem er die Tasche 30 der Schablone 26 verwendet. Da sich sowohl der erste Röntgenfilm als auch der zweite Röntgenfilm 38 bei der Bildaufnahme relativ zu dem Zahn 20 an der gleichen Position befanden, sind deren jeweiligen Röntgenbilder gut vergleichbar. Das ganze kann der Facharzt oder auch der überweisende Arzt mehrfach wiederholen, um den Heilungsfortschritt zu begutachten. Da die intraorale-Röntgenaufnahme als Projektion stets exakt gleich aufgenommen wurde, ist die Beurteilung des Heilungsverlauf um ein Vielfaches einfacher und schneller möglich als sie es wäre, wenn die Röntgenfilme 38 aus unterschiedlichen Positionen miteinander verglichen werden müssten.

Hierbei ist die Tasche 30 der Bohrschablone 26 als Nebenprodukt der eigentlichen Behandlung abgefallen, ohne dass der Facharzt großen zusätzlichen Arbeitsaufwand gehabt hätte. Es versteht sich, dass die Erfindung nicht nur auf Röntgenfilme 38 beschränkt ist, sondern dass vielmehr jegliche Sensormittel, welche zur Aufnahme im Mundraum geeignet sind, unter den Offenbarungsgehalt der Erfindung fallen.

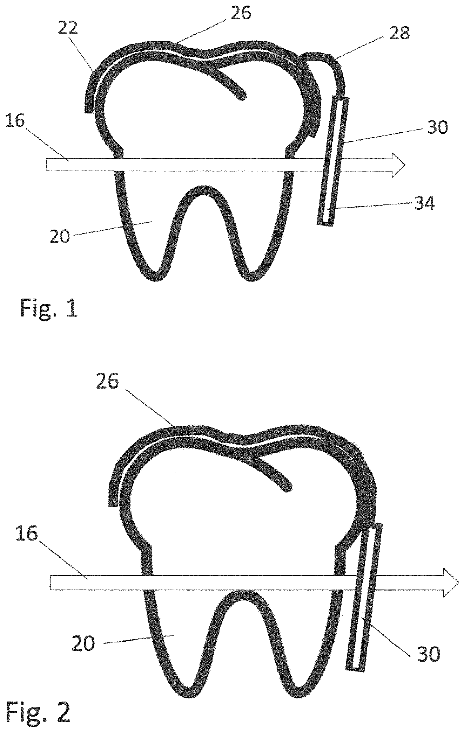

Fig. 1 zeigt ein erstes schematisches Ausführungsbeispiel der erfindungsgemäßen Bohrschablone 26 in einer Schnittdarstellung. Ein Abschnitt der Bohrschablone 26, der auf den Zahn 20 aufgesetzt wird, ist hierbei derart an eine Zahnoberfläche 22 des Zahns 20 angepasst, dass die Bohrschablone 26 lösbar an dem Zahn 20 fixiert ist. Von dem zahngetragenen Abschnitt der Bohrschablone 26 geht ein Brückenelement 28 ab an welchem die Tasche 30 zur Aufnahme des Röntgenfilms 38 angeschlossen ist. Durch das Brückenelement 28 können auf einfache Weise verschiedene räumliche Positionen der Tasche 30 bei der Herstellung realisiert werden. Zudem ist in Fig. 1 eine Durchstrahlrichtung 16 gezeigt, die die Vorzugsrichtung der Röntgenstrahlung bei der Bildgebung wiedergibt. Vor der Bildaufnahme wird der zweite Röntgenfilm 38 in die Tasche 30 eingebracht, wobei die Position der Tasche 30 die Lage des ersten Röntgenfilms repliziert, sodass sich der zweite Röntgenfilm 38 relativ zum Zahn 20 in derselben Position wie der erste Röntgenfilm befindet.

Fig. 2 zeigt ein zweites Ausführungsbeispiel der Bohrschablone 26, wobei die Tasche 30 direkt und ohne das Brückenelement 28 an die Bohrschablone 26 angeschlossen ist. Bevorzugt werden die Bohrschablone 26 und die Tasche 30 sowohl in dem ersten Ausführungsbeispiel als einem zweiten Ausführungsbeispiel einteilig gefertigt.

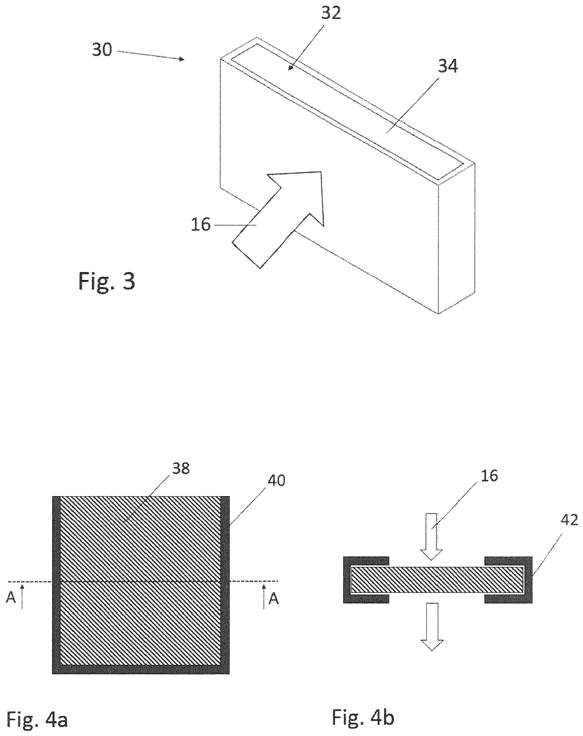

Fig. 3 zeigt ein erstes Ausführungsbeispiel der Tasche 30 der Bohrschablone 26 zur Aufnahme von Röntgenfilmen in perspektivischer Darstellung. Im Wesentlichen entspricht die Form der Tasche 30 einem rechteckigen Hohlkörper mit einer Öffnung 32 in die der Röntgenfilm 38 einbringbar ist. Bevorzugt ist die Öffnung 32 auf einer Oberseite angeordnet, wobei unter der Oberseite die Seite zu verstehen ist, die der Gravitationsrichtung entgegen gerichtet ist. Auf diese Weise wird verhindert, dass der Röntgenfilm 38 unabsichtlich durch die Gravitation aus der Tasche 30 herausfällt, nachdem er in einen Aufnahmeraum 34 eingebracht wurde. Die Tasche 30 besteht aus biokompatiblen Material.

Fig. 4a zeigt ein zweites Ausführungsbeispie! des Halteelements 30 der Bohrschablone 26 aus Fig. 3 in einem Querschnitt entlang der Durchstrahlrichtung 16 der Röntgenstrahlung. Die Seitenflächen des Halteelements 30 in Durchstrahlrichtung 16 sind nunmehr nicht mehr, wie in Fig. 3 gezeigt, flächig aus Material ausgebildet, sondern das Material weist in der Durchstrahlrichtung 16 die Form eines U-förmlichen Rahmens 40 auf. Dies hat den Vorteil, dass die Röntgenstrahlung den im Aufnahmeraum 34 eingebrachten Röntgenfilm 38 durchqueren kann ohne von dem Material des Halteelements 30 abgeschwächt zu werden.

Fig. 4b zeigt das Halteelement 30 in einem Schnitt durch die Ebene A aus Fig. 4a. Hierbei ist gezeigt, dass Seitenaufnahmen 42 des Halteelements 30 ebenfalls in einem Querschnitt U-förmig ausgebildet sind, sodass der Röntgenfilm 38 einfach in das Halteelement 30 eingeschoben werden kann.

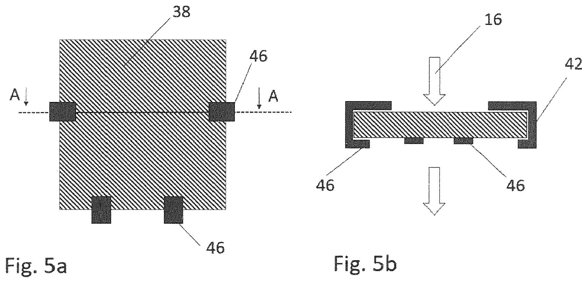

Fig. 5a zeigt ein drittes Ausführungsbeispiel des Halteelements 30 der Bohrschablone 26 aus Fig. 3 in einem Querschnitt entlang der Durchstrahlrichtung 16 der Röntgenstrahlung. In diesem Ausführungsbeispiel ist das Material des Halteelements 30 weiter reduziert, sodass der Röntgenfilm 38 mittels Haltehaken 46 gehalten ist. Im Prinzip ist die Anzahl der Haltehaken 46 frei wählbar, solange ein "sicheres" Halten des Röntgenfilms 38 gewährleistet ist.

Fig. 5b zeigt das Halteelement 30 inklusive der Haltehaken 46 in einer Draufsicht durch die Ebene A aus Fig. 5a. In Fig. 5b ist gezeigt, dass die Haltehaken 46 mit der Seitenaufnahme 42 verbunden sind.

## Patentansprüche

1. Verfahren zur Herstellung einer Haltevorrichtung für Röntgenfilme an Zähnen eines Patienten, wobei
mittels eines Rechners eine Bildregistrierung eines ersten zweidimensionalen Röntgenbildes eines Zahns (20) des Unter- oder Oberkiefers in einem dreidimensionalen volumentomographischen Datensatz des Kiefers durchgeführt wird, wobei
die Lage des ersten Röntgenbildes innerhalb des volumentomographischen Datensatzes bestimmt wird,
wobei anhand des volumentomographischen Datensatzes ein virtuelles Modell für eine an den Zähnen (20) des Unter- oder Oberkiefers formschlüssig anbringbare Haltevorrichtung (26) erstellt wird, die ein Halteelement (30) für die Halterung eines Röntgenfilms (38) in definierter Position aufweist, wobei
die definierte Position durch die Lage des ersten Röntgenbilds im volumentomographischen Datensatz festgelegt wird und
wobei die Haltevorrichtung (26) entsprechend dem virtuellen Modell hergestellt wird.

2. Verfahren nach Anspruch 1,
**dadurch gekennzeichnet, dass**
die Haltevorrichtung (26) mittels einer Frästechnik und/oder im 3D-Druck anhand der definierten Position im volumentomographischen Datensatz hergestellt wird.

3. Verfahren nach einem der vorherigen Ansprüche,
**dadurch gekennzeichnet, dass** die Haltevorrichtung (26) aus biokompatiblen Material ist.

4. Verfahren nach einem der vorherigen Ansprüche,
**dadurch gekennzeichnet, dass** die Haltevorrichtung (26), insbesondere das Haltelement (30), aus einem homogenen Material mit einer geringeren Ordnungszahl hergestellt wird.

5. Verfahren nach einem der vorherigen Ansprüche,
**dadurch gekennzeichnet, dass** das Halteelement (30) in einer Durchstrahlrichtung (16) von Röntgenstrahlung dünner als 1 mm ausgebildet ist.

6. Verfahren nach einem der Ansprüche 1 bis 5,
**dadurch gekennzeichnet, dass** das Halteelement (30) in einer Durchstrahlrichtung (16) von Röntgenstrahlung in einer U-Form ausgebildet ist.

7. Verfahren nach einem der vorherigen Ansprüche,
**dadurch gekennzeichnet, dass** die geometrischen Abmessungen des Röntgenfilms (38) bei der Herstellung des Halteelements (30) eingerechnet werden.

8. Haltevorrichtung für Röntgenfilme an Zähnen eines Patienten mit einem Halteelement (30) für die Halterung des Röntgenfilms (38) in definierter Position, hergestellt mit einem Verfahren nach einem der Ansprüche 1 bis 7.

9. Haltevorrichtung nach Anspruch 8,
**dadurch gekennzeichnet, dass** der Röntgenfilm (38) formschlüssig in das Halteelement (30) einsteckbar ist.

10. Haltevorrichtung nach einem der Ansprüche 8 bis 9,
**dadurch gekennzeichnet, dass** die Haltevorrichtung (26) einen Zahnaufsatz aufweist.

11. Haltevorrichtung nach einem der Ansprüche 8 bis 10,
**dadurch gekennzeichnet, dass** der Zahnaufsatz mittels eines Brückenelements (28) mit dem Halteelement (30) verbunden ist.

## Claims

1. Method for producing a holding apparatus for x-ray films of a patient's teeth, wherein
an image registration of a first two-dimensional x-ray image of a tooth (20) in the lower or upper jaw is performed in a three-dimensional volume-tomographic dataset of the jaw by means of a computer, wherein the location of the first x-ray image within the volume-tomographic dataset is determined,
wherein a virtual model for a holding apparatus (26) that is attachable in form-fitting manner to the teeth (20) of the lower or upper jaw is prepared on the basis of the volume-tomographic dataset, which holding apparatus includes a holding element (30) for holding an x-ray film (38) in a defined position, wherein
the defined position is established by the location of the first two-dimensional x-ray image in the three-dimensional volume-tomographic dataset, and
wherein the holding apparatus (26) is produced on the basis of the virtual model.

2. Method according to Claim 1,
**characterized in that**
the holding apparatus (26) is produced by means of a milling process and/or by 3D-printing based on the defined position in the volume-tomographic dataset.

3. Method according to either one of the preceding claims,
**characterized in that** the holding apparatus (26) is made from biocompatible material.

4. Method according to any one of the preceding claims,
**characterized in that** the holding apparatus (26), particularly the holding element (30), is produced from a homogenous material which has a low atomic number.

5. Method according to any one of the preceding claims,
**characterized in that** the holding element (30) is designed with a thickness less than 1 mm in a projection direction (16) of x-ray radiation.

6. Method according to any one of Claims 1 to 5,
**characterized in that** the holding element (30) is designed in a U-shape in a projection direction (16) of x-ray radiation.

7. Method according to any one of the preceding claims,
**characterized in that** the geometric dimensions of the x-ray film (38) are taken into consideration when producing the holding element (30).

8. Holding apparatus for x-ray films of a patient's teeth, with a holding element (30) for holding the x-ray film (38) in a defined position, produced by a method according to any one of Claims 1 to 7.

9. Holding apparatus according to Claim 8,
**characterized in that** the x-ray film (38) is insertable in the holding element (30) form-fitting manner.

10. Holding apparatus according to any of Claims 8 to 9,
**characterized in that** the holding apparatus (26) includes a tooth attachment.

11. Holding apparatus according to any one of Claims 8 to 10,
**characterized in that** the tooth attachment is connected to the holding element (30) via a bridge element (28).

## Revendications

1. Procédé de fabrication d'un dispositif de maintien pour des films radiographiques sur les dents d'un patient, sachant
qu'un enregistrement d'image d'une première image radiographique bidimensionnelle d'une dent (20) de la mâchoire inférieure ou supérieure est exécuté dans un ensemble de données tomographique volumique de la mâchoire, sachant que
la position de la première image radiographique est déterminée à l'intérieur de l'ensemble de données tomographique volumique,
sachant qu'un modèle virtuel est établi à l'aide de l'ensemble de données tomographique volumique pour un dispositif de maintien (26) applicable par conformité de forme aux dents (20) de la mâchoire inférieure ou supérieure, qui comporte un élément de maintien (30) pour la fixation d'un film radiographique (38) dans une position définie, sachant que
la position définie est définie par le positionnement de la première image radiographique dans l'ensemble de données tomographique volumique et
sachant que le dispositif de maintien (26) est fabriqué selon le modèle virtuel.

2. Procédé selon la revendication 1,
**caractérisé en ce que**
le dispositif de maintien (26) est fabriqué au moyen d'une technique de fraisage et/ou en impression tridimensionnelle à l'aide de la position définie dans l'ensemble de données tomographique volumique.

3. Procédé selon l'une quelconque des revendications précédentes,
**caractérisé en ce que** le dispositif de maintien (26) est en matériau biocompatible.

4. Procédé selon l'une quelconque des revendications précédentes,
**caractérisé en ce que** le dispositif de maintien (26), en particulier l'élément de maintien (30), est fabriqué dans un matériau homogène avec un nombre atomique plus faible..

5. Procédé l'une quelconque des revendications précédentes,
**caractérisé en ce que** l'élément de maintien (30) est constitué plus fin qu'1 mm dans une direction de transmission de rayonnement (16) du rayonnement radiographique.

6. Procédé selon l'une quelconque des revendications 1 à 5,
**caractérisé en ce que** l'élément de maintien (30) est constitué en forme de U dans une direction de transmission du rayonnement (16) du rayonnement radiographique.

7. Procédé selon l'une quelconque des revendications précédentes,
**caractérisé en ce que** les dimensions géométriques du film radiographique (38) sont prises en compte lors de la fabrication de l'élément de maintien (30).

8. Dispositif de maintien pour des films radiographiques sur des dents d'un patient avec un élément de maintien (30) pour la fixation du film radiographique (38) dans une position définie, réalisé avec un procédé selon l'une quelconque des revendications 1 à 7.

9. Dispositif de maintien selon la revendication 8, **caractérisé en ce que** le film radiographique (38) peut être insérée dans l'élément de maintien (30) par conformité de forme.

10. Dispositif de maintien selon l'une quelconque des revendications 8 à 9,
**caractérisé en ce que** le dispositif de maintien (26) comporte une bague dentaire.

11. Dispositif de maintien selon l'une quelconque des revendications 8 à 10,
**caractérisé en ce que** la bague dentaire est reliée à l'élément de maintien (30) au moyen d'un élément de pontage (28).
